Europäisches Patentamt

European Patent Office

Office européen des brevets

(11) Veröffentlichungsnummer : **0 385 112 B1**

(12) **EUROPÄISCHE PATENTSCHRIFT**

(19)

(45) Veröffentlichungstag der Patentschrift :
**05.08.92 Patentblatt 92/32**

(21) Anmeldenummer : **90101650.1**

(22) Anmeldetag : **27.01.90**

(51) Int. Cl.$^5$ : **A61F 5/01**

(54) **Cervicalstütze.**

(30) Priorität : **28.02.89 DE 3906233**

(43) Veröffentlichungstag der Anmeldung :
**05.09.90 Patentblatt 90/36**

(45) Bekanntmachung des Hinweises auf die
Patenterteilung :
**05.08.92 Patentblatt 92/32**

(84) Benannte Vertragsstaaten :
**AT BE CH DE DK ES FR GB GR IT LI LU NL SE**

(56) Entgegenhaltungen :
**DE-A- 2 404 683**
**DE-U- 8 510 523**
**FR-A- 2 507 887**
**US-A- 3 964 474**
**US-A- 4 401 111**

(73) Patentinhaber : **ADEV, GESELLSCHAFT FÜR
ENTWICKLUNG UND VERTRIEB VON
MEDIZINTECHNISCHEN ARTIKELN MBH
Grapengiesserstrasse 21
W-2400 Lübeck (DE)**

(72) Erfinder : **Grundei, Jaana
Hamburger Strasse 89
W-2400 Lübeck (DE)**
Erfinder : **Timmermann, Andreas
Birkenweg 11
W-2360 Klein Rönnau (DE)**

(74) Vertreter : **Weiss, Christian, Dipl.-Ing. et al
Dr. Fuchs, Dr. Luderschmidt, Dipl.-Phys. Seids,
Dr. Mehler, Patentanwälte,
Abraham-Lincoln-Strasse 7
W-6200 Wiesbaden (DE)**

EP 0 385 112 B1

## Beschreibung

Eine Cervicalstütze dieser Art ist in der DE-A-24 04 683 beschrieben. Obwohl sich diese bekannte Cervicalstütze in der Praxis gut bewährt hat, wurde festgestellt, daß ihre Paßform an verschiedenen Abstützungsbereichen verbesserungsbedürftig ist, insbesondere am vorderen Halsbereich, um einen nach längerem Tragen als unangenehm empfundenen Druck an den als Abstützungsfläche dienenden Körperpartien zu vermindern oder zu vermeiden.

Die Aufgabe der Erfindung besteht in der Verbesserung einer Cervicalstütze der einleitend angeführten Art dahingehend, daß einerseits eine medizinisch notwendige Immobilisierung der Halswirbelsäule anatomiegerecht, sicher und schnell erreicht wird und daß andererseits der Tragekomfort der Cervicalstütze gesteigert ist.

Die Lösung der Aufgabe gelingt mit der Cervikalstütze mit den Merkmalen des Anspruchs 1.

Vorteilhafte Weiterbildungen ergeben sich aus den Unteransprüchen.

Im Nackenstützbereich ist die Cervicalstütze so ausgebildet, daß sie eine obere Randaussparung aufweist, deren Grund tiefer liegt als das Niveau der seitlich angrenzenden Teile des Nackenstützbereiches. Weiterhin kann der untere Rand des Nackenstützbereiches in bezug auf den unteren Rand des vorderen Kinnstützbereiches beträchtlich tiefer nach unten versetzt sein.

Die erfindungsgemäße Cervicalstütze hat eine verbesserte Paßform, die das Tragen der Cervicalstütze, insbesondere wenn diese über eine längere Zeit getragen werden muß, wesentlich angenehmer macht und andererseits eine anatomiegerechtere Immobilisierung der Halswirbelsäule gestattet. Durch den nicht mehr ovalförmigen Umfangsverlauf der Cervicalstütze und durch die veränderten Aufstützbereiche des oberen und des unteren Umfangsrandes der Cervicalstütze werden nunmehr auch weitere Körperpartien des Patienten zur Abstützungsfunktion herangezogen, so daß die von der Cervicalstütze ausgehenden und auf den Patienten wirkenden Druckwirkungen flächenmäßig besser verteilt sind und den Patienten daher entlasten. Dies wirkt sich insbesondere wohltuend auf den vorderen Halsbereich und auf den Hinterkopfbereich und den halsnahen Rückenbereich des Patienten aus. Des weiteren wird auch eine verbesserte Kopfbeweglichkeit des Patienten erzielt, ohne daß die Immobilisierung der Halswirbelsäule verschlechtert wird, was den Tragekomfort der Cervicalstütze weiter erhöht. Die somit noch anatomiegerechtere erfindungsgemäße Cervicalstütze erlaubt ferner eine Erweiterung des diesbezüglichen Indikationskataloges um zum Beispiel die Anwendung bei frischen Knochenfrakturen und Wirbeltumoren und kann nun auch von solchen Patienten getragen werden, die im Wirkungsbereich der Cervicalstütze besonders vorspringende Skeletteile haben.

Die erfindungsgemäße Cervicalstütze ist nachstehend anhand eines in den anliegenden Zeichnungen dargestellten Ausführungsbeispieles näher erläutert. Es zeigen:

Figur 1 das an einen Patienten angelegte Ausführungsbeispiel im Längsschnitt,

Figur 2 das Ausführungsbeispiel in Aufsicht,

Figur 3 das Ausführungsbeispiel allein im Längsschnitt,

Figur 4 das Ausführungsbeispiel in Vorderansicht gemäß dem Pfeil P.

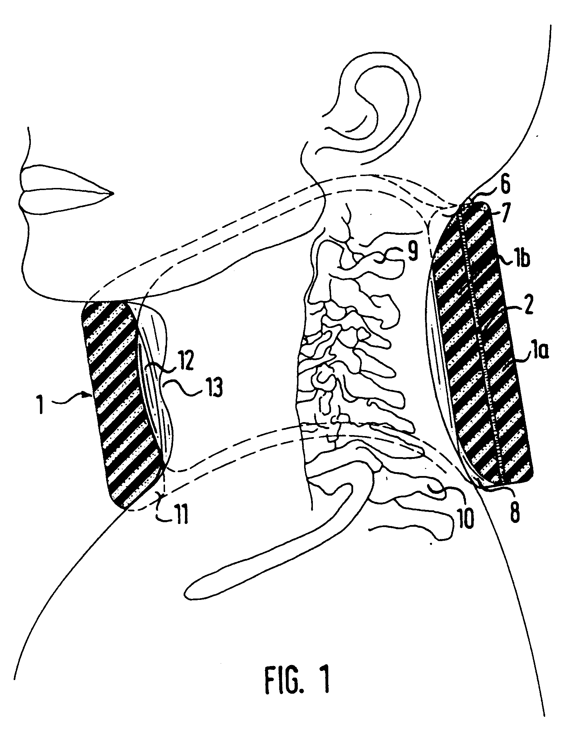

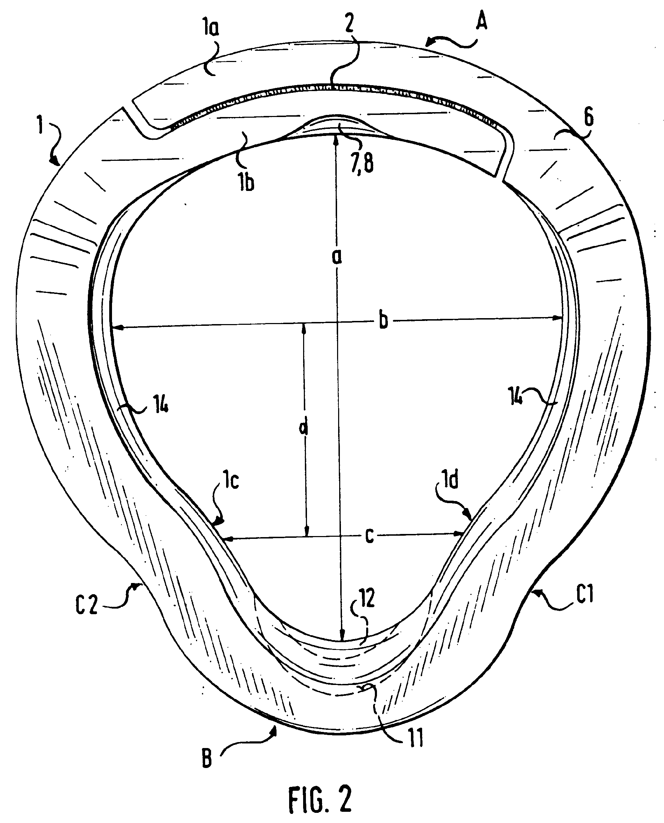

Die aus einem flexiblen und formstabilen Stützkörper 1 aus elastischem Schaumstoff bestehende Cervicalstütze liegt gemäß Figur 1 eng an dem Hals eines Patienten an und wird im Nackenbereich an den sich überlappenden Endteilen 1a, 1b (Figur 2) mittels eines Verschlusses 2, vorzugsweise mittels eines sogenannten Klettverschlusses, zusammengehalten.

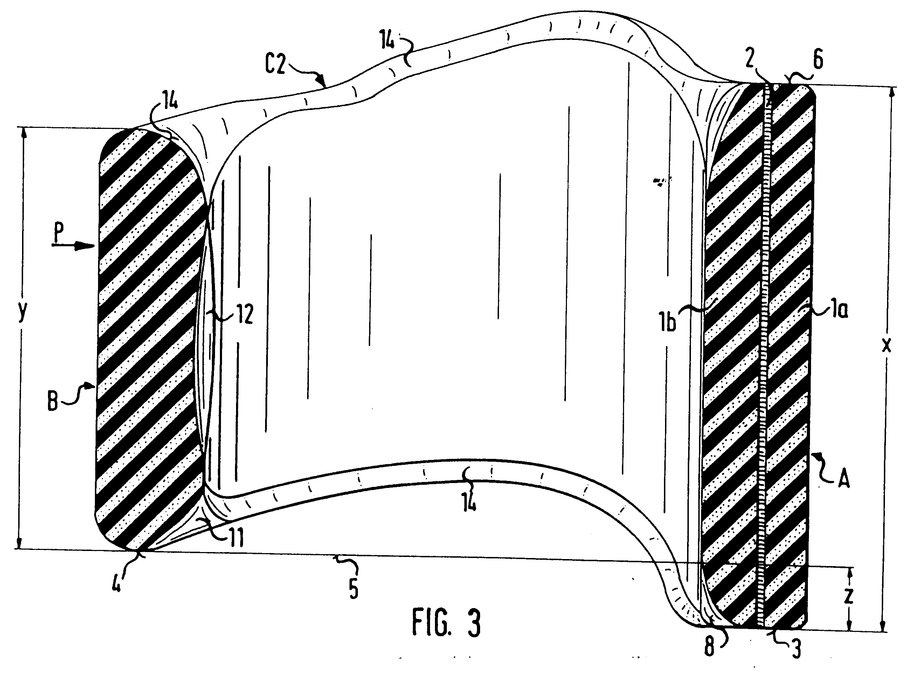

Der Stützkörper 1 ist an seinem oberen und an seinem unteren Umfangsrand im wesentlichen den anatomischen Gegebenheiten des an den Hals angrenzenden Kopf- und Schulterbereiches des Patienten angepaßt. Dabei weist der Nackenstützbereich A des Körpers 1 eine größere Höhe auf als der Kinnstützbereich B, wobei die Nackenstützhöhe $x$ um etwa das Maß 1,15 bis 1,70, vorzugsweise 1,15 bis 1,40, größer ist als die Kinnstützhöhe $y$. Des weiteren ist der untere Rand 3 des Nackenstützbereiches A gegenüber dem unteren Rand 4 des Kinnstützbereiches B tiefer nach unten verlegt ausgebildet, wie es am besten aus Figur 3 zu erkennen ist. Das Versetzungsmaß $z$ beträgt etwa 0,15 bis 0,25, vorzugsweise 0,18 bis 0,22, des Wertes der Nackenstützhöhe $x$. Als Bezugslinie für das Maß $z$ wird eine horizontale, an den unteren Rand 4 des Kinnstützbereiches B gelegte Tangente 5 gewählt, von der aus sich das Maß $z$ nach unten erstreckt. Durch diese Tieferlegung des Nackenstützbereiches liegt die Cervicalstütze entsprechend weiter nach unten verlagert an dem Patienten an, was zu einer angenehmeren Druckverteilung beim Tragen der Stütze in diesem Körperaufstützbereich führt.

Zur Verbesserung der Anlage der Cervicalstütze am Hals des Patienten weist die Stütze eine geänderte Umfangsform auf, die aus Figur 2 am besten zu erkennen ist. Man erkennt daraus, daß der Nackenstützbereich A einen teilelliptischen Verlauf hat, der sich etwa bis zu einer ersten Querachse $b$ erstreckt. Alternativ ist auch ein teilkreisförmiger Verlauf dieses Bereiches A möglich. An den Bereich A schließen sich beidseitig einer Längsachse $a$, die durch die Mitten der Bereiche A und B verläuft, zwei zur Längsachse $a$ spiegelbildlich symmetrisch verlaufende Kieferstützbereiche C1 und C2 an, die je nach innen, also zur Längsachse $a$ hin gerichtet, gewölbt ausgebildet sind und somit gewisse Bögen 1c, 1d bilden. Diese Bögen haben ihre innenliegenden Scheitel etwa im Bereich einer weiteren Querachse c, die zur ersten Querachse b parallel verläuft. Im Kinnstützbereich B ist die Krümmung wieder etwa kreisbogenförmig ausgeführt.

Wenn man die Länge der Längsachse a als Bezugsgröße wählt, dann beträgt die Länge der ersten Querachse b etwa 0,80 bis 0,95 vom Wert der Länge der Achse a und die Länge der weiteren, kleineren Querachse c beträgt etwa 0,5 bis 0,7 vom Wert der Länge der Achse a. Beide Querachsen haben einen Abstand d voneinander, der dem Wert 0,35 bis 0,45 und vorzugsweise 0,40 bis 0,43 des Wertes der Länge der Achse a entspricht, und verlaufen senkrecht zu der Längsachse a.

Durch die flache Bogenform der Kieferstützbereiche C1 und C2 wird eine wesentlich verbesserte Stützung der Halswirbelsäule im vorderen Halsbereich erzielt.

Eine weitere Verbesserung der Cervicalstütze besteht darin, daß der Oberrand des Nackenstützbereiches A tiefer gelegt ist, wie Figur 3 am besten zeigt. Hierdurch wird erreicht, daß der Reaktionsdruck der Cervicalstütze gegen den Hinterhauptbeinhöcker gemindert oder vermieden wird. Die Tieferlegung des Oberrandes des Nackenstützbereiches ist beträchtlich, so daß eine ausgeprägte Aussparung 6 gegeben ist, deren Grund tiefer liegt als das Niveau der seitlichen Teile des Nackenstützbereiches. Obwohl es vorgezogen wird, den gesamten Grund der Randaussparung vertieft anzuordnen, kann auch so vorgegangen sein, daß nur der innere Überlappungsendteil 1b der Cervicalstütze mit der Aussparung 6 versehen ist. Des weiteren ist es möglich, daß die Randaussparung 6 zwar an beiden Endteilen 1a, 1b vorgesehen ist, daß aber der hintere Teil 1a eine höher liegende Aussparung aufweist als der vordere Teil 1b.

Ferner ist der hintere innere Endteil 1b oben und unten sowie im Bereich der Längsmitte der Cervicalstütze, also im Verlauf der Längsachse a, mit inneren Ausnehmungen 7 und 8 versehen, die ebenfalls eine Druckentlastung bewirken. Diese Ausnehmungen haben die Form von relativ kurzen, zu den Rändern des Teiles Ib hin offenen Auskehlungen und ermöglichen, daß bei einigen Patienten die von dem obersten Halswirbel 9 und von dem obersten Brustwirbel 10 ausgehenden Druckbelästigungen besonders gemildert werden (Figur 1).

Ebenfalls ist auch der Kinnstützbereich B vorne unten mit einer inneren mittigen Ausbuchtung 11 versehen, um Druck auf das Brustbeinoberende und die Brustbein-Schlüsselbeingelenke zu mindern oder zu vermeiden.

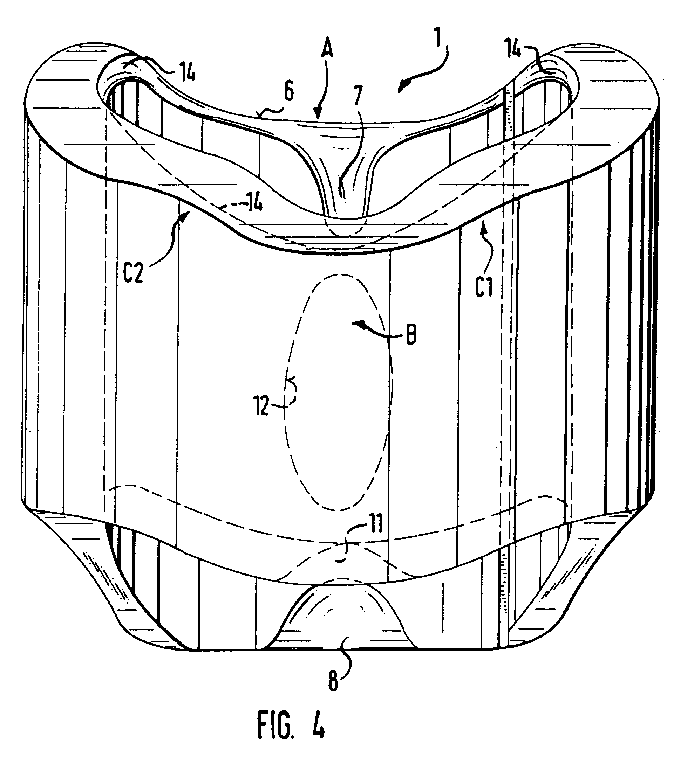

Des weiteren ist der Kinnstützbereich B an der Innenseite mit einer mittigen, etwa ovalförmigen Mulde 12 versehen, die einen Freiraum für den Kehlkopf 13 des Patienten bildet, wie die Figuren 1, 3 und 4 am besten zeigen.

Um die Anlage der Cervicalstütze am Körper des Patienten und das Tragen der Cervicalstütze noch angenehmer zu machen, sind der ohnehin gerundete obere und untere Rand der Cervicalstütze innenseitig und im wesentlichen auf dem gesamten Umfangsverlauf noch stärker abgetragen, wie es besonders mit 14 in Figur 3 zum Ausdruck kommt. Auch hierdurch wird eine bessere, das heißt weniger merkbare Druckbelastung im Kantenbereich der Cervicalstütze erreicht.

Das Material für die Stütze 1 besteht in bekannter Weise aus einem flexiblen Schaumstoff, der mit einem üblichen Schutzüberzug (nicht gezeigt) aus Textil- oder Kunststoffmaterial überzogen ist. An der so fertiggestellten Stütze ist im Überlappungsbereich 1a, 1b, der vorteilhaft im Nackenbereich liegt, der bereits erwähnte Klettverschluß 2 angebracht, mit dem ein stufenloses Regulieren der Anlagefestigkeit am Hals als auch bei unterschiedlichem Halsdurchmesser möglich ist. Es ist jedoch auch möglich, andere Verschlüsse zu verwenden.

## Patentansprüche

1. Cervicalstütze, bestehend aus einem formstabilen, mit einem Überzug aus Textilmaterial versehenen und um den Hals des Patienten legbaren Stützkörper (1) aus elastischem Schaumstoff, dessen freie Enden (1a, 1b) sich hinten am Nackenstützbereich (A) überlappen und lösbar miteinander verbindbar sind, wobei die Höhe (x) des Nackenstützbereiches größer ist als die Höhe (y) des diesem gegenüberliegenden vorderen Kinnstützbereiches (B) des Stützkörpers und wobei die beiden seitlichen Kieferstützbereiche (C1, C2) hinsichtlich ihrer oberen und unteren Randkonturen der anatomischen Form des Kinnes und halsnahen Körperbereiches angepaßt sind,
dadurch gekennzeichnet,
daß, jeweils in Aufsicht gesehen, der Nackenstützbereich (A) einen teilkreisförmigen oder teilelliptischen Verlauf hat, der an einer ersten, senkrecht auf einer die Mitten des Nackenstützbereiches (A) und des Kinnstützbereiches (B) verbindenden Längsachse (a) stehenden Querachse (b) in die Kieferstützbereiche (C1, C2) übergeht, die in bezug auf die Längsachse (a) spiegelbildlich symmetrisch verlaufen mit einem zur Längsachse (a) hin gerichteten Bogen (1c, 1d) mit innenliegendem Scheitel, deren Verbindung eine zweite Querachse (c) bildet, die senkrecht zur Längsachse (a) steht und von der ersten Querachse (b) in Richtung auf den Kinnstützbereich (B) beabstandet ist und parallel zu dieser verläuft,

daß der Kinnstützbereich (B) einen teilkreisförmigen Verlauf aufweist, und

daß die Längen der Achsen (a, b, c) sich verhalten wie a : b : c = 1 : 0,8 bis 0,95 : 0,5 bis 0,7.

2. Cervicalstütze nach Anspruch 1, dadurch gekennzeichnet, daß die Querachsen (b) und (c) einen Abstand (d) voneinander haben, der 0,35 bis 0,45 des Wertes der Länge der Längsachse (a) beträgt.

3. Cervicalstütze nach Anspruch 2, dadurch gekennzeichnet, daß die Querachsen (b) und (c) einen Abstand (d) voneinander haben, der 0,40 bis 0,43 des Wertes der Länge der Längsachse (a) beträgt.

4. Cervicalstütze nach einem der Ansprüche 1 bis 3, dadurch gekennzeichnet, daß für die vorerwähnten Höhen (x,y) folgende Beziehung gilt, nämlich 1,15y < x <1,4y.

5. Cervicalstütze nach einem der Ansprüche 1 bis 4, dadurch gekennzeichnet, daß der untere Rand des Nackenstützbereiches (A) in bezug auf den unteren Rand des vorderen Kinnstützbereiches (B) um das Maß (z) 0,15 bis 0,25 des Wertes der vorgenannten Höhe (x) tiefer nach unten versetzt ist.

6. Cervicalstütze nach einem der Ansprüche 1 bis 5, dadurch gekennzeichnet, daß der untere Rand des Nackenstützbereiches (A) in bezug auf den unteren Rand des vorderen Kinnstützbereiches (B) um das Maß (z) 0,18 bis 0,22 des Wertes der vorgenannten Höhe (x) tiefer nach unten versetzt ist.

7. Cervicalstütze nach einem der Ansprüche 1 bis 6, dadurch gekennzeichnet, daß im Nackenstützbereich (A) oben eine Randaussparung (6) vorgesehen ist, deren Grund tiefer liegt als das Niveau der seitlich angrenzenden Teile des Nackenstützbereiches.

8. Cervicalstütze nach Anspruch 7, dadurch gekennzeichnet, daß die Randaussparung (6) nur am innen liegenden Abschnitt (1b) der sich überlappenden Stützkörperenden (1a, 1b) vorgesehen ist, während der obere Rand des außen befindlichen Abschnittes (1a) dazu auf einem höherem Niveau liegt.

9. Cervicalstütze nach einem der Ansprüche 1 bis 8, dadurch gekennzeichnet, daß der vordere untere Rand des Kinnstützbereiches (B) eine Ausbuchtung (11) aufweist.

10. Cervicalstütze nach einem der Ansprüche 1 bis 9, dadurch gekennzeichnet, daß mittig an der Innenseite des Kinnstützbereiches (B) eine Mulde (12) als Kehlkopf-Freiraum vorgesehen ist.

11. Cervicalstütze nach einem der Ansprüche 1 bis 10, dadurch gekennzeichnet, daß sie auf der Innenseite des Nackenstützbereiches (A) eine mittige obere Ausnehmung (7) und eine mittige untere Ausnehmung (8) aufweist.

## Claims

1. A cervical collar comprising a dimensionally stable supporting body (1) provided with a protective layer of textile material wrapped around the neck of a patient and made of elastic foamed material and the free ends thereof (1a, 1b) being overlapping at the neck support section (A) at the back being enabled to be detachably connected, the altitude (x) of the neck support section being larger than the altitude (y) of the chin support section (B) of the supporting body opposite to the said neck support section and, with respect to the upper and lower outer rim contours, the two lateral support sections (C1, C2) being adjusted to the anatomic form of the chin and to the portion of the body being close to the neck,

characterized in that,

each taken in top view, the neck support section (A) shows a limb-shaped or sub-elliptical course that verges into the jaw support section (C1, C2) at a first lateral axis (b) standing vertically to a longitudinal axis which connects the centers of the neck support sections (A) and the chin support section (B), the said jaw support sections (C1, C2) running axially symmetric with respect to the longitudinal axis (a) and having an arc (1c, 1d) with internal vertex pointing in the direction of the longitudinal axis, the connection of the jaw support sections forming a second lateral axis (c) standing vertically to the longitudinal axis (a) at the distance from the first lateral axis (b) in the direction of the chin support section (B) and running in parallel to the said first lateral axis (b),

in that the chin support section (B) comprises a limb-shaped course, and

in that the ratio of the lengths of the axes (a, b, c) a: b: c is equal to that of 1: 0,8 through 0,95 : 0,5 through 0,7.

2. A cervical collar according to claim 1, characterized in that the lateral axes (b) and (c) have a distance (1) which amounts to 0,35 through 0,45 of the value of the length of the longitudinal axis (a).

3. A cervical collar according to claim 2, characterized in that the lateral axes (b) and (c) have a distance (d) which amounts to 0,40 through 0,43 of the value of the length of the longitudinal axis (a).

4. A cervical collar according to one of claims 1 through 3, characterized in that for the above-mentioned altitudes (x, y) stands the following relation, viz. 1,15y < x < 1,4y.

5. A cervical collar according to one of claims 1 through 4, characterized in that, with respect to the lower rim of the front chin support section (B), the lower rim of the neck support section (A) is displaced more deeply downwards by the measure (z) 0,15 through 0,25 of the value of the previously stated altitude (x).

6. A cervical collar according to one of claims 1 through 5, characterized in that, with respect to the lower rim of the front chin support section (B), the lower rim of the neck support section (A) is displaced more deeply downwards by the measure (z) 0,18 through 0,25 of the value of the previously stated altitude (x).

7. A cervical collar according to one of claims 1 through 6, characterized in that in the neck support section (A) a rim recess (6) is provided on the top, the bottom of the said recess being situated deeper than the level of the portions adjoining at the sides of the neck support section.

8. A cervical collar according to claim 7, characterized in that the rim recess (6) is only provided at the inside portion (1b) of the overlapping ends of the supporting bodies (1a, 1b), whilst the top rim of the exterior portion (1a) lies on a higher level.

9. A cervical collar according to one of claims 1 through 8, characterized in that the lower front rim of the chin support section (B) comprises a curved projection (11).

10. A cervical collar according to one of claims 1 through 9, characterized in that, in the dead centre at the inside of the chin support section (B), a depression (12) is provided as a clearance for the larynx.

11. A cervical collar according to one of claims 1 through 10, characterized in that it comprises a centric upper recess (7) and a centric lower recess (8) at the inside of the neck support section (A).


## Revendications

1. Minerve, constituée d'un corps de soutien (1) indéformable, pourvue d'un revêtement textile et à placer autour du cou du patient, réalisée dans une mousse élastique dont les extrémités libres (1a, 1b) se recouvrent derrière la zone de soutien du cou (A) et peuvent être reliées l'une à l'autre de manière amovible, la hauteur (x) de la zone de soutien du cou étant supérieure à la hauteur (y) de la zone de soutien du menton (B) avant, faisant face à la première, du corps de soutien et les deux zones latérales de soutien des mâchoires (C1, C2) étant adaptées par leurs contours de bord supérieur et inférieur à la forme anatomique du menton et à la zone du corps voisine du cou,

caractérisée en ce que,

vue de dessus, la zone de soutien du cou (A) a une forme en partie circulaire ou en partie elliptique qui se prolonge, sur un premier axe transversal (b) perpendiculaire à un axe longitudinal (a) reliant les centres de la zone de soutien du cou (A) et de la zone de soutien du menton (B), par les zones de soutien des mâchoires (C1, C2) qui sont symétriques en miroir par rapport à l'axe longitudinal (a), avec un arc (1c, 1d), dirigé vers l'axe longitudinal (a), avec sommet situé à l'intérieur, dont la liaison forme un deuxième axe transversal (c) qui est perpendiculaire à l'axe longitudinal (a) et qui est espacé du premier axe transversal (b), en direction de la zone de soutien du menton (B) et parallèle à celui-ci,

en ce que la zone de soutien du menton (B) a une forme en partie circulaire et

en ce que les longueurs des axes (a, b, c) sont telles que a : b : c = 1 : 0,8 à 0,95 : 0,5 à 0,7.

2. Minerve selon la revendication 1, caractérisée en ce que les axes transversaux (b) et (c) sont espacés l'un de l'autre d'une distance (d) qui est comprise entre 0,35 et 0,45 de la valeur de la longueur de l'axe longitudinal (a).

3. Minerve selon la revendication 2, caractérisée en ce que les axes transversaux (b) et (c) sont espacés l'un de l'autre d'une distance (d) qui est comprise entre 0,40 et 0,43 de la valeur de la longueur de l'axe longitudinal (a).

4. Minerve selon l'une des revendications 1 à 3, caractérisée en ce que pour les hauteurs précitées (x, y) on a la relation suivante, à savoir 1,15 y < x < 1,4 y.

5. Minerve selon l'une des revendications 1 à 4, caractérisée en ce que le bord inférieur de la zone de soutien du cou (A) est décalé plus profondément vers le bas, par rapport au bord inférieur de la zone de soutien du menton (B) inférieure, d'une distance (z) comprise entre 0,15 et 0,25 de la valeur de la hauteur (x) précitée.

6. Minerve selon l'une des revendications 1 à 5, caractérisée en ce que le bord inférieur de la zone de soutien du cou (A) est décalé plus profondément vers le bas, par rapport au bord inférieur de la zone de soutien du menton (B) avant, de la distance (z) comprise entre 0,18 et 0,22 de la valeur de la hauteur (x) précitée.

7. Minerve selon l'une des revendications 1 à 6, caractérisée en ce qu'il est prévu, dans la zone de soutien du cou (A), à sa partie supérieure, une découpe de bord (6) dont le fond se situe plus bas que le niveau des parties latérales adjacentes de la zone de soutien du cou.

8. Minerve selon la revendication 7, caractérisée en ce que la découpe de bord (6) n'est prévue que sur la portion (1b) intérieure des extrémités du corps de soutien (1a, 1b) se recouvrant l'une l'autre, tandis que le bord supérieur de la portion (1a) située à l'extérieur, se trouve à un niveau plus haut.

9. Minerve selon l'une des revendications 1 à 8, caractérisée en ce que le bord inférieur avant de la zone de soutien du menton (B) présente un renflement (11).

10. Minerve selon l'une des revendications 1 à 9, caractérisée en ce qu'il est prévu une gorge (12) sous la forme d'un espace libre de larynx, au milieu du côté intérieur de la zone de soutien du menton (B).

11. Minerve selon l'une des revendications 1 à 10, caractérisée en ce qu'elle présente, sur le côté intérieur de la zone de soutien du cou (A), un évidement (7) supérieur central et un évidement (8) inférieur central.

FIG. 1

FIG. 2

FIG. 3

EP 0 385 112 B1

FIG. 4

10